# EUROPEAN PATENT APPLICATION

(11) **EP 2 128 262 A1**
(43) Date of publication of application: **02.12.2009**
(21) Application number: 08009693.6
(22) Date of filing: 28.05.2008
(51) Int. Cl.: C12P 7/40, C12P 7/56, C12N 1/16

(54) **Improved yeast strains for organic acid production**

(71) Applicant: Università Degli Studi Di Milano - Bicocca, 20126 Milano (IT)
(72) Inventor: Porro, Danilo, 22036 Erba (CO) (IT); Branduardi, Paola, 20134 Milano (IT); Sauer, Michael, 1040 Wien (AT)
(74) Representative: Minoja, Fabrizio

(57) **Abstract**

The present invention relates to the production of organic acids with yeasts that overexpress at least one sugar transporter. The yeast might express further genes related to the production of the desired organic acid. The organic acid is produced by cultivation of the yeast overexpressing a sugar transporter in an adequate culture medium, whereupon the desired organic acid is accumulated in the culture medium and subsequently purified to the desired degree by techniques known in the art.

## Description

The present invention relates to the production of organic acids with yeast. More in particular the present invention relates to yeasts producing organic acids and to processes for the production of organic acids by means of yeasts that overexpress one or more hexose transporters.

### BACKGROUND OF THE INVENTION

A variety of organic acids are widely used in the food and pharmaceutical industries and draw more and more attention as new building block materials for the chemical industry (Werpy, T. and G. Petersen, Top Value Added Chemicals From Biomass. 2004, US Department of Energy: Oak Ridge TN). Particularly, if produced by environmentally benign fermentation strategies they provide a sound alternative to petroleum derived and therefore limited building block materials. Examples of such organic acids include lactic, citric, itaconic and succinic acid.

The microorganisms which are used for such processes include bacteria, filamentous fungi and yeasts. However, such processes suffer from a number of drawbacks resulting in particular in long fermentation periods required and high costs of purification of the acids due a low product concentration combined with high concentration of impurities.

Costs are the crucial factor determining if such a process can be a true alternative for building block production. Consequently, methods are sought to increase the productivity of organic acid production with microorganisms. One field to increase productivity is to enhance the robustness of the production strains.

For example, a culture medium having a low pH is favourable for the production of organic acids, as thereby the free acid is produced rather than the anionic form, what generally reduces costs of purification. However, such a production environment exerts a high stress on the microorganisms: the culture medium is acidified, so that the cells have to actively counteract the increased pH gradient across the plasma membrane. At low external pH, organic acids exert additional stress on the cells, as they diffuse through the plasma membrane and acidify the cytoplasm. Given this stress, there is a limitation of productivity by using state of the art technology, as the microbial cells will eventually lose viability and metabolic activity.

One possibility to counteract this effect is the isolation of more robust microbial strains, i.e., strains capable of improved viability and metabolic activity at low pH. Methods for such selection or sorting strategies have been proposed, for example US 20050112737 and US 20070065899.

In fact, many approaches to increase productivity rely on the assumption that the production environment (like acid stress) limits the productivity as outlined before. Another main focus for strain improvement relates to the product formation rate itself, in the sense that the enzymatic activities being involved in product formation are enhanced.

Examples for enzymatic activities which are typically enhanced include the central metabolic pathways as glycolysis, which very often provide the required intermediates for the production of organic acids.

However, even recognizing that in yeast, such as *Saccharomyces cerevisiae* one determining step in glycolytic flux regulation is the sugar uptake, i.e. transportation of the sugar across the cell membrane, this fact has never been conceived to improve productivity of organic acid production in microorganisms.

The present invention relates to the increase of microbial productivity of organic acids by an increase of the hexose uptake.

It has been shown that CO₂ and ethanol production can be improved by overexpression of hexose transporters in *S. cerevisiae* (EP0785275, US6159725, Perez et al. 2005, Guillaume et al. 2007, and Gutiérrez-Lomelí et al. 2008).

However, an influence of the hexose uptake on organic acid production with microorganisms was never described.

It appears implausible that sugar uptake could be a limiting factor for microbial organic acid production, particular at low pH, but surprisingly, the overexpression of a hexose transporter in a yeast strain can improve its organic acid productivity as is outlined below.

In baker's yeast 20 genes are known up to now, that encode proteins similar to glucose (hexose) transporters or related to hexose uptake (HXT1 to HXT17, GAL2, SNF3, and RGT2). Many and different studies were done to determine the respective features of these transporters (affinity and capacity), as well as to construct strains deleted in one or more HXT genes, and in different combinations and finally to construct chimera proteins combining affinity and capacity of different transporters.

### SUMMARY OF THE INVENTION

It is an objective of the present invention to provide a method for the production of organic acids with yeasts that overexpress at least one hexose transporter.

The overexpression of at least one hexose transporter increases the productivity of the organic acid in respect to a yeast strain not overexpressing the hexose transporter.

The yeast strain might express further genes related to the production of the desired organic acid. The organic acid is produced by cultivation of the yeast overexpressing a hexose transporter in an adequate culture medium, whereupon the desired organic acid is accumulated in the culture medium and subsequently purified to the desired degree by techniques known in the art.

Moreover, we demonstrate that the increase of the glycolytic flux determined by the hexose transporter overexpression can have a positive effect not only in respect to microbial productivity, but also on biomass accumulation. Said phenomenon can be more or less evident in respect to the yeast background.

### BRIEF DESCRIPTION OF THE DRAWINGS

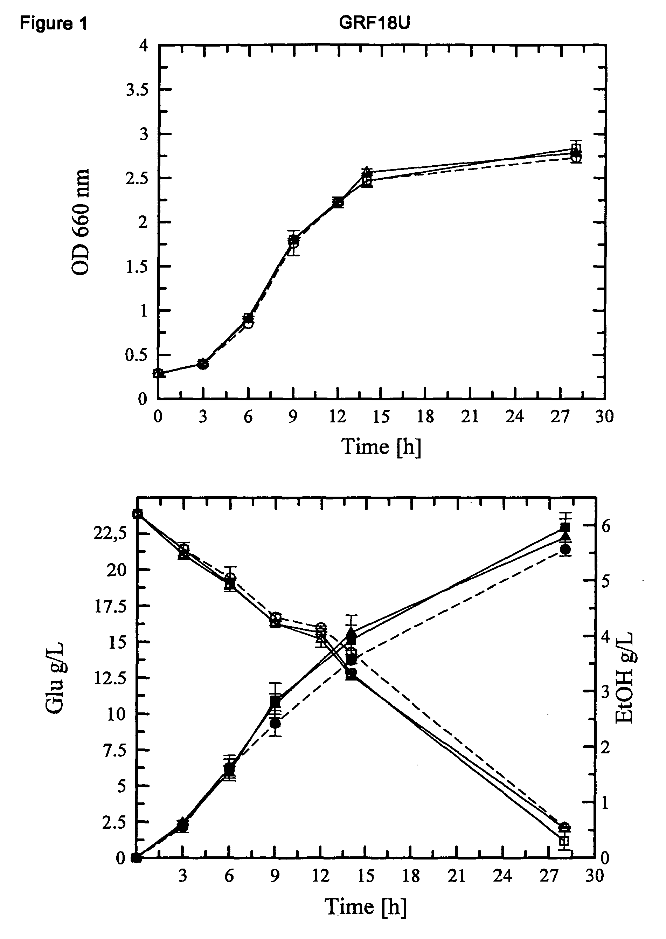

**Figure 1****:** (Over)expression of *HXT1* or *HXT7* genes in the *S. cerevisiae* GRF18U strain.

Growth kinetics in minimal (YNB) medium, with glucose 2% (w/V) as a carbon source. On the upper panel, growth was measured as optical density (OD 660nm) for control (circles, dotted line), *HXT1* (squares, full line) and *HXT7* (triangles, full line) transformants. On the bottom panel, for the corresponding strains, glucose consumption (open symbols) and ethanol production (full symbols) were determined. The data correspond to the mean values of three independent clones independently tested at least twice.

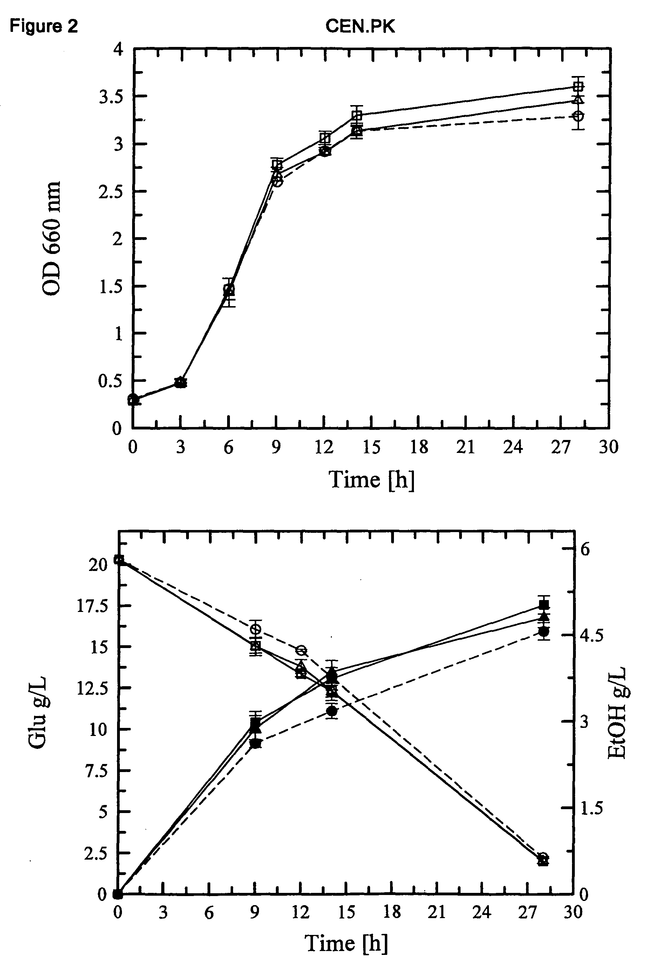

**Figure 2****:** (Over)expression of *HXT1* or *HXT7* genes in the *S. cerevisiae* CEN.PK strain.

Growth kinetics in minimal (YNB) medium, with glucose 2% (w/V) as a carbon source. On the left, growth was measured as optical density (OD 660nm) for control (circles, dotted line), *HXT1* (squares, full line) and *HXT7* (triangles, full line) transformants. On the right, for the corresponding strains, glucose consumption (open symbols) and ethanol production (full symbols) were determined. The data correspond to the mean values of three independent clones independently tested at least twice.

**Figure 3****:** (Over)expression of *HXT1* or *HXT7* genes in the *S. cerevisiae* GRF18U (left panel) or CEN.PK (right panel) strain: effect on ethanol and biomass accumulation
The increase (expressed as a percentage) of biomass accumulation and ethanol production calculated on the maximum accumulation/production of the kinetics described in Figures 1 and 2 of the transformants in respect to the control strain is given.

**Figure 4****.****1****:** Lactic acid and ethanol production in a GRF18U *LpLDH* transformed *S. cerevisiae* strain (over)expressing the *HXT1* (open symbols).

Growth kinetics in minimal (YNB) medium, with glucose 5% (w/V) as a carbon source.

**a)** cellular growth was measured as optical density (OD 660nm) for control (circles, dotted line) and *HXT1* (squares, full line) transformants.

For the corresponding strains, glucose consumption **(b),** ethanol **(c)** and lactic acid **(d)** productions were determined.

The data correspond to the mean values of three independent clones independently tested at least twice.

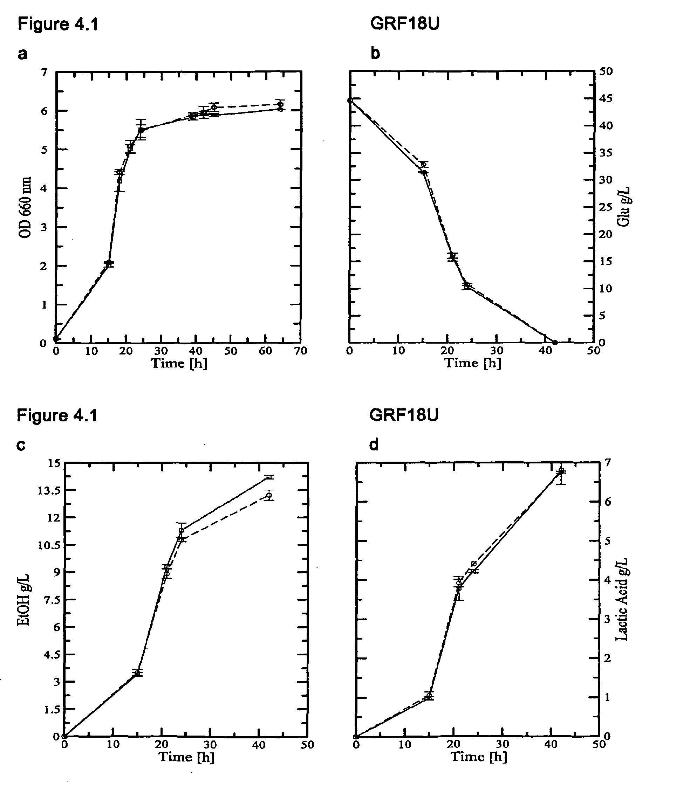

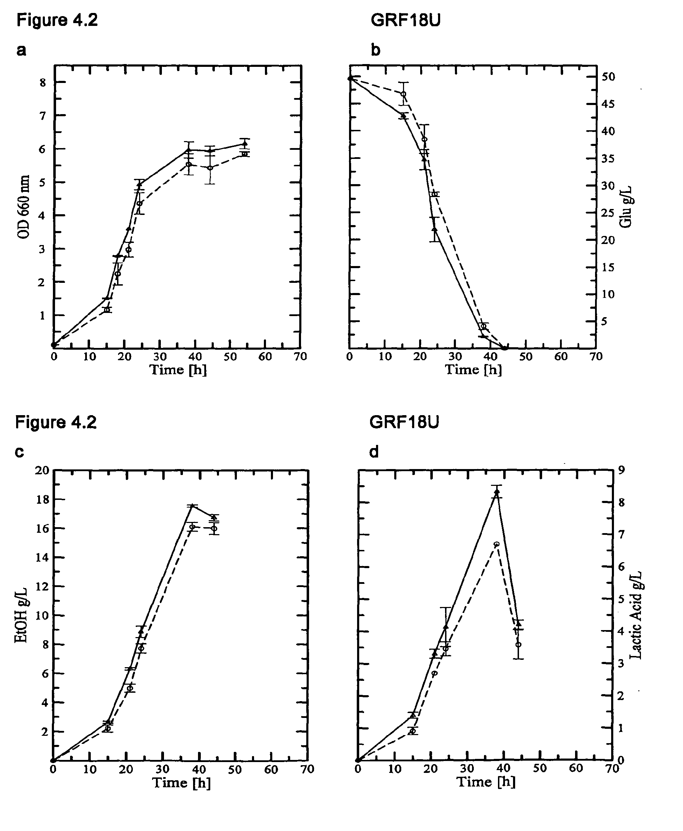

**Figure 4****.****2****:** Lactic acid and ethanol production in a GRF18U *LpLDH* transformed *S. cerevisiae* strain (over)expressing the *HXT7* (open symbols).

Growth kinetics in minimal (YNB) medium, with glucose 5% (w/V) as a carbon source.

### a) cellular growth was measured as optical density (OD 660 nm) for control (circles, dotted line) and HXT7 (triangles, full line) transformants.

For the corresponding strains, glucose consumption **(b),** ethanol **(c)** and lactic acid **(d)** productions were determined.
The data correspond to the mean values of three independent clones independently tested at least twice.

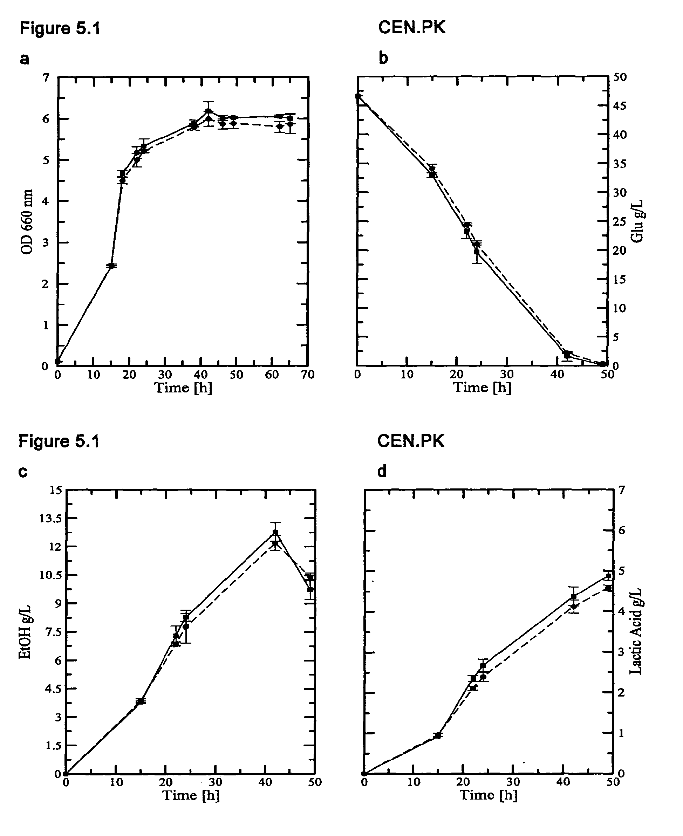

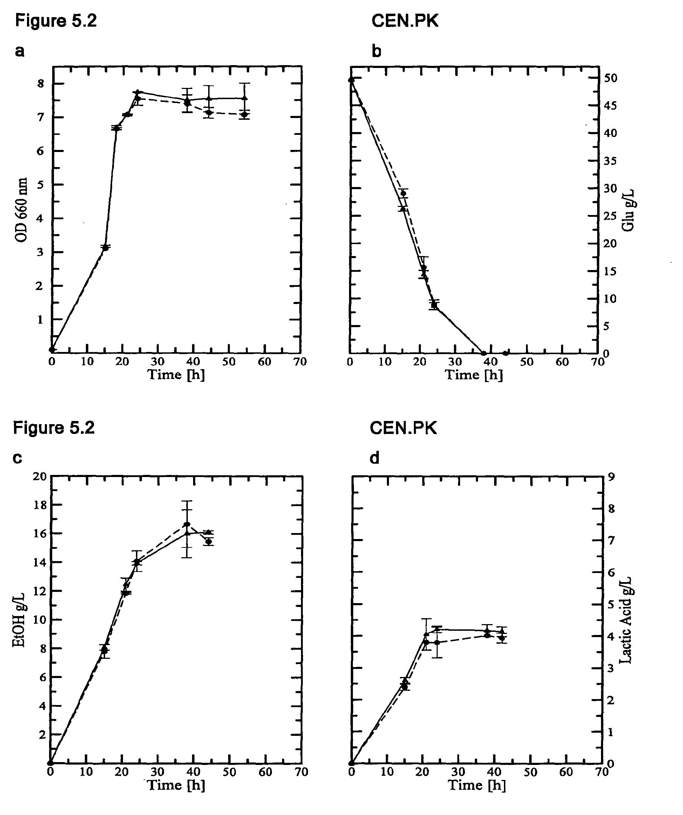

**Figure 5****.****1****:** Lactic acid and ethanol production in a CEN.PK *LpLDH* transformed *S. cerevisiae* strain (over)expressing the *HXT1* (full symbols).

Growth kinetics in minimal (YNB) medium, with glucose 5% (w/V) as a carbon source.

**a)** cellular growth was measured as optical density (OD 660nm) for control (circles, dotted line), *HXT1* (squares, full line) transformants.

For the corresponding strains, glucose consumption **(b),** ethanol **(c)** and lactic acid **(d)** productions were determined.

The data correspond to the mean values of three independent clones independently tested at least twice.

**Figure 5****.****2****:** Lactic acid and ethanol production in a CEN.PK *LpLDH* transformed *S. cerevisiae* strain (over)expressing the *HXT7* (full symbols).
Growth kinetics in minimal (YNB) medium, with glucose 5% (w/V) as a carbon source.

**a)** cellular growth was measured as optical density (OD 660nm) for control (circles, dotted line), *HXT7* (triangles, full line) transformants.

For the corresponding strains, glucose consumption (**b**), ethanol (**c**) and lactic acid **(d)** productions were determined.

The data correspond to the mean values of three independent clones independently tested at least twice.

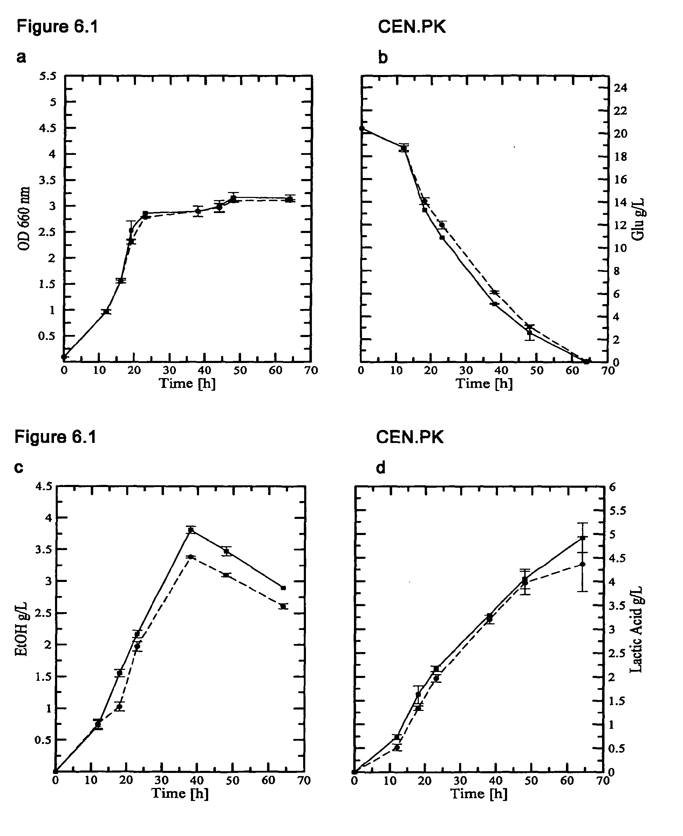

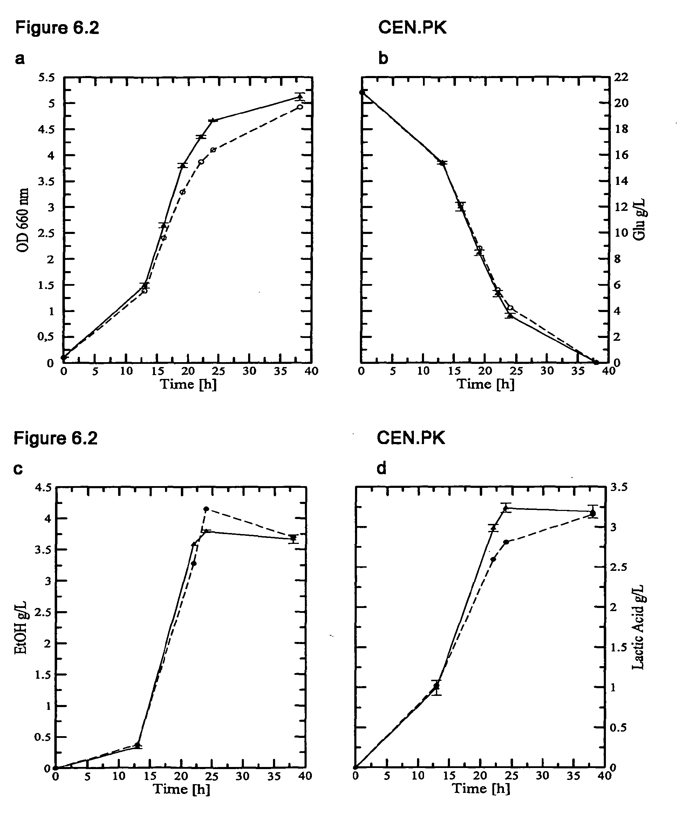

**Figure 6****.****1****:** CEN.PK *S. cerevisiae* strain (over)expressing the *HXT1* gene and the *LpLDH* gene in a multicopy plasmid: ethanol and lactic acid production (full symbols).

Growth kinetics in minimal (YNB) medium, with glucose 5% (w/V) as a carbon source.

**a)** cellular growth was measured as optical density (OD 660nm) for control (circles, dotted line), and *HXT1* (squares, full line) transformants.

For the corresponding strains, glucose consumption (**b**), ethanol (**c**) and lactic acid (**d**) productions were determined.

The data correspond to the mean values of three independent clones independently tested at least twice.

**Figure 6****.****2****:** CEN.PK *S. cerevisiae* strain (over)expressing the *HXT7* gene and the *LpLDH* gene in a multicopy plasmid: ethanol and lactic acid production (full symbols).

Growth kinetics in minimal (YNB) medium, with glucose 5% (w/V) as a carbon source.

**a)** cellular growth was measured as optical density (OD 660nm) for control (circles, dotted line), and *HXT7* (triangles, full line) transformants.

For the corresponding strains, glucose consumption (**b**), ethanol (**c**) and lactic acid (**d**) productions were determined.

The data correspond to the mean values of three independent clones independently tested at least twice.

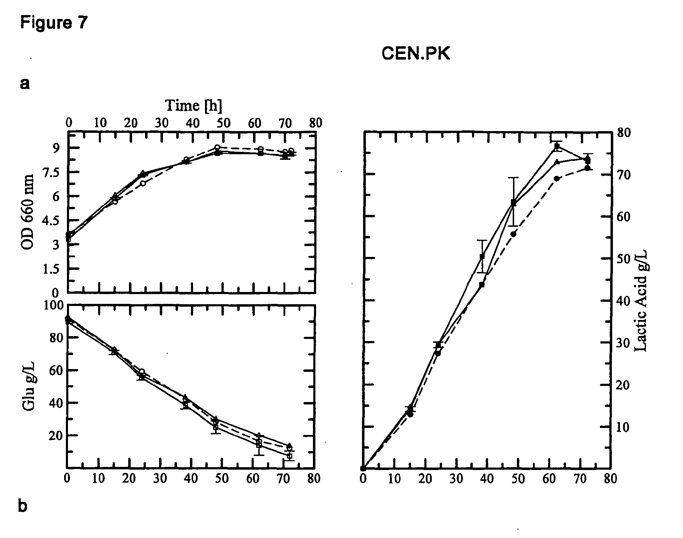

**Figure 7****:** Lactic acid production in the m850 (*S. cerevisiae* CEN.PK, *pdc⁻, LpLDH* overexpressing) strain (over)expressing the *HXT1* or the *HXT7* gene.

Growth kinetics were performed at 28°C in 250-ml quadruple baffled shake flasks in minimal medium containing 4.5 g/l CaCO₃, 1.7 g/I YNB without amino acids and without (NH₄)₂SO₄, 1 g/l urea, 5 g/l ethanol, and with glucose 9% (w/V) as a carbon source.

a) cellular growth was measured as optical density (OD 660nm, open symbols) for control (circles, dotted line), *HXT1* (squares, full line) and *HXT7* (triangles, full line) transformants.

For the corresponding strains, glucose consumption (**b**, open symbols) and lactic acid (c, full symbols) productions were determined.

### DESCRIPTION OF ILLUSTRATIVE EMBODIMENTS

In one embodiment, the present invention relates to a method of preparing an organic acid, comprising:
a. obtaining a recombinant yeast with increased hexose transporter activity;
b. culturing the recombinant yeast in a medium comprising a hexose, thereby forming the organic acid, and
c. isolating the organic acid.

The present invention refers therefore to a method of producing an organic acid by incubating a yeast strain overexpressing at least one gene related to hexose uptake in a medium comprising a carbon source, to produce the organic acid. Another term for "incubating the yeast strain in an appropriate medium" is "culturing" the yeast strain. The yeast might grow under the chosen condition that means the amount of biomass as measured for example by the optical density at 660 nm, increases. In another embodiment the yeast strain does not grow under the chosen condition, that means the biomass does not increase, but the organic acid is produced anyhow.

Increase of the hexose transport activity means that the rate of translocation of hexoses across the plasma membrane is increased. This can be achieved by overexpression of at least one hexose transporter. Hexose transporters are therefore proteins capable of transporting hexoses. An increase of the overall hexose transporter activity can be accomplished by increasing the expression of the homologous hexose transporter genes or by introduction of further copies of homologous or heterologous hexose transporter genes. An increase of the hexose transporter activity can also be achieved by expression or overexpression of a gene regulating the hexose transport activity, preferably the gene stimulates the hexose transport activity. It shall be noted that it is also within the scope of the invention to increase the capacity of the hexose transporters instead of an increase in expression.

The organic acid that is produced can be selected from the list of acrylic acid, lactic acid, itaconic acid, succinic acid, fumaric acid, malic acid, 2,5-furan dicarboxylic acid, 3-hydroxy propionic acid, aspartic acid, citric acid, glucaric acid, glutamic acid, malonic acid, propionic acid, gluconic acid, kojic acid, comeric acid, ascorbic acid, adipic acid, levulinic acid or any other desired organic acid.

In a preferred embodiment of the invention the yeast strain overexpresses additionally to the hexose transporter at least one gene related to the production of the organic acid. For example, in one embodiment the yeast strain expresses the hexose transporter and a heterologous lactic acid dehydrogenase gene to enable the yeast cells to produce lactic acid. The yeast strain might comprise further genetic modifications beneficial for the organic acid production. These might be introduced by means of genetic recombination techniques or random mutagenesis or other techniques known in the art. For example, the afore mentioned yeast strain for production of lactic acid has preferably a decreased pyruvate decarboxylase activity, even more preferred is the knock out of any pyruvate decarboxylase activity.
The yeast strain used in the present invention for the production of an organic acid can be selected from any known genus and species of yeasts. Yeasts are described for example by N. J. W. Kreger-van Rij, "The Yeasts," Vol. 1 of Biology of Yeasts, Ch. 2, A. H. Rose and J. S. Harrison, Eds. Academic Press, London, 1987. In one embodiment the yeast genus can be *Saccharomyces, Zygosaccharomyces, Candida, Hansenula, Kluyveromyces, Debaromyces, Nadsonia, Lipomyces, Torulopsis, Kloeckera, Pichia, Schizosaccharomyces, Trigonopsis, Brettanomyces, Cryptococcus, Trichosporon, Aureobasidium, Lipomyces, Phaffia, Rhodotorula, Yarrowia*, or *Schwanniomyces*, among others. Preferably the yeast is selected from *Saccharomyces, Zygosaccharomyces*, or *Kluyveromyces*. More preferably the yeasts are *S. cerevisiae, Z. bailii*, or *K. lactis*. In a particularly preferred embodiment, the yeast is *S. cerevisiae* strain GRF18U, W3031 B, BY4742 (EuroScarf Accession No. Y10000), CEN.PK strains 102-5B *(MATa, ura3-52*, *his3-11, leu2-3*/*112, TRP1, MAL2-8c, SUC2*) and 113-11C (*MATa, ura3-52, his3-11, TRP1, MAL2-8c, SUC2 -* Dr. P. Kötter, Institute of Microbiology, Johann Wolfgang Goethe-University, Frankfurt, Germany), CEN.PK RWB837 *(MATa pdc1::loxP pdc5::loxP pdc6::loxP ura3-52)* (van Maris *et al*., 2004), CEN.PK RWB876 and CEN.PK m850 (Liu and Lievense 2005) or *Z. bailii* ATCC 60483; or *K. lactis* PM6-7A.

The yeast maybe haploid or diploid.

The coding region encoding the hexose transporter gene to be overexpressed in the yeast strain of the invention can be isolated from any source. In one embodiment, the coding region of the hexose transporter is isolated from *Saccharomyces cerevisiae.* It should be noted that a coding region is "isolated" from an organism if it encodes a protein sequence substantially identical to that of the same protein purified from cells of the organism.

Preferably, a coding region encoding the hexose transporter is incorporated into the yeast in such a manner that the desired hexose transporter is produced in the yeast and is substantially functional. Such a yeast may be referred to herein as being "functionally transformed."

A functional hexose transporter facilitates the uptake of hexoses. In other words, hexose transporters stimulate the translocation of hexoses across the plasma membrane.

Once the coding region has been extracted from an organism's nucleic acids ("was isolated") or synthesized by chemical means, it can be prepared for transformation into and expression in the yeast. At minimum, this involves the insertion of the coding region into a vector and operable linkage to a promoter found on the vector and active in the yeast. Any vector (integrative, chromosomal or episomal) can be used.

Any promoter active in the target host (homologous or heterologous; constitutive, inducible or repressible) can be used. Such insertion can involve the use of restriction endonucleases to "open up" the vector at a desired point where operable linkage to the promoter is possible, followed by ligation of the coding region into the desired point. If desired, before insertion into the vector, the coding region can be prepared for use in the target organism. This can involve altering the codons used in the coding region to more fully match the codon use of the target organism; changing sequences in the coding region that could impair the transcription or translation of the coding region or the stability of an mRNA transcript of the coding region; or adding or removing portions encoding signaling peptides (regions of the protein encoded by the coding region that direct the protein to specific locations (e.g. an organelle, the membrane of the cell or an organelle, or extracellular secretion)), among other possible preparations known in the art.

Regardless whether the coding region is modified, when the coding region is inserted into the vector, it is operably linked to a promoter active in the yeast. A promoter, as is known, is a DNA sequence that can direct the transcription of a nearby coding region. As already described, the promoter can be constitutive, inducible or repressible. Constitutive promoters continually direct the transcription of a nearby coding region. Inducible promoters can be induced by the addition to the medium of an appropriate inducer molecule, which will be determined by the identity of the promoter. Repressible promoters can be repressed by the addition to the medium of an appropriate repressor molecule, which will be determined by the identity of the promoter. In one embodiment, the promoter is constitutive. In a further embodiment, the constitutive promoter is the *S. cerevisiae* triosephosphateisomerase (TPI) promoter.

The vector comprising the coding region operably linked to the promoter can be a plasmid, a cosmid, or a yeast artificial chromosome, among others known in the art to be appropriate for use in yeast. In addition to the coding region operably linked to the promoter, the vector can also comprise other genetic elements. For example, if the vector is not expected to integrate into the yeast genome, the vector can comprise an origin of replication, which allows the vector to be passed on to progeny cells of a yeast comprising the vector (e.g. 2µ derived or ARS/CEN). If integration of the vector into the yeast genome is desired, the vector can comprise sequences homologous to sequences found in the yeast genome, and can also comprise coding regions that can facilitate integration. To determine which yeast cells are transformed, the vector can comprise a selectable marker or screenable marker which imparts a phenotype to the yeast that distinguishes it from untransformed yeast, e.g. it survives on a medium comprising an antibiotic fatal to untransformed yeast or it metabolizes a component of the medium into a product that the untransformed yeast does not, among other phenotypes. In addition, the vector may comprise other genetic elements, such as restriction endonuclease sites and others typically found in vectors.

After the vector is prepared, with the coding region operably linked to the promoter, the yeast can be transformed with the vector (i.e. the vector can be introduced into at least one of the cells of a yeast population). Techniques for yeast transformation are well established, and include electroporation, microprojectile bombardment, and the LiAc/ssDNA/PEG method, among others. Yeast cells, which are transformed, can then be detected by the use of a screenable or selectable marker on the vector. It should be noted that the phrase "transformed yeast" has essentially the same meaning as "recombinant yeast". The transformed yeast can be one that received the vector in a transformation technique, or can be a progeny of such a yeast.

If the hexose transporter is a homologous protein to the host cell, i.e. a protein which is naturally occurring in the host cell, the expression of the hexose transporter in the host cell may be modulated by the exchange of its native promoter sequence with another promoter sequence functional in the host cell.

This purpose may be achieved e.g. by transformation of a host cell with a recombinant DNA molecule comprising homologous sequences of the target gene to allow site specific recombination, the desired yeast promoter sequence and a selective marker suitable for the host cell. The site specific recombination shall take place in order to operably link the (recombinant) yeast promoter sequence with the nucleotide sequence encoding the hexose transporter. This results in the expression of the hexose transporter from the (recombinant) yeast promoter sequence instead of from the native promoter sequence. The selected yeast promoter may have an increased promoter activity relative to the native promoter sequence leading to an increased expression of the hexose transporter in the host cell.

After a recombinant yeast has been obtained, the yeast can be cultured in a medium. The medium in which the yeast can be cultured can be any medium known in the art to be suitable for this purpose. Culturing techniques and media are well known in the art. In one embodiment, culturing can be performed by aqueous fermentation in an appropriate vessel. Examples for a typical vessel for yeast fermentation comprise a shake flask or a bioreactor.

The production process for the organic acid by the yeast can be performed as chemostat, batch culture or as fed-batch culture, or any other known type of cultivation.

In a preferred embodiment the cultivation is performed at low pH to facilitate the purification of the organic acid. Preferably the pH of the culture medium at the beginning or the end of the cultivation or both is less than 4, even more preferably the pH is less than 3, and even more preferably the pH is less than 2.5.

The medium can comprise D-glucose or other saccharides (sugars) as hexoses or pentoses. It can further comprise any other component required for the growth of the yeast. D-glucose can be a component required for the growth of the yeast but need not be, it could be just the precursor for organic acid production. Preferably, the concentration of the saccharide being the precursor for the organic acid production is high in the beginning of the cultivation and low in the end. Preferably, the saccharide concentration is greater than 70 g/l in the beginning, even more preferably greater than 100 g/l, even more preferably greater than 150 g/l. Preferably, the saccharide is completely consumed during the cultivation, so the preferred concentration at the end of the cultivation is less than 10 g/l, preferably less then 5 g/l, even more preferably less then 1 g/l.

The medium can comprise a carbon source other than D-glucose, such as sucrose, fructose, lactose, D-galactose, or hydrolysates of vegetable matter, among others. In one embodiment, the medium can also comprise a nitrogen source as either an organic or an inorganic molecule. In a further embodiment, the medium can also comprise components such as amino acids; purines; pyrimidines; corn steep liquor; yeast extract; protein hydrolysates; water-soluble vitamins, such as B complex vitamins; or inorganic salts such as chlorides, hydrochlorides, phosphates, or sulfates of Ca, Mg, Na, K, Fe, Ni, Co, Cu, Mn, Mo, or Zn, among others. Further components known to one of ordinary skill in the art to be useful in yeast culturing or fermentation can also be included. The medium can be buffered but need not be.

During the fermentation, the D-glucose is internalized by the yeast and converted, through a number of steps, into the organic acid. The produced organic acid can be collected within the yeast, or can be accumulated in the medium, by secretion or other processes that lead to the translocation of the organic acid through the plasma membrane.

Minimal media are preferred for organic acid production according to the invention; a particularly preferred medium comprises D-glucose and YNB.

After culturing has progressed for a sufficient length of time to produce a desired concentration of the organic acid, the latter can be isolated. "Isolated," as used herein with reference to the organic acid, means being brought to a state of greater purity by separation of the organic acid from at least one other component of the yeast or the medium. Preferably, the isolated organic acid is at least about 90% pure, more preferably about 95% pure, even more preferably at least about 99% pure.

The first step of isolation of an organic acid after the yeast is separated from the medium, can be lysing of the yeast by chemical or enzymatic treatment, treatment with glass beads, sonication, freeze/thaw cycling, or other known techniques. The organic acid can be purified from the membrane, protein, and nucleic acid fractions of the yeast lysate by appropriate techniques, such as centrifugation, filtration, microfiltration, ultrafiltration, nanofiltration, liquid-liquid extraction, crystallization, enzymatic treatment with nuclease or protease, or chromatography, among others.

The isolation of an organic acid accumulated in the medium can comprise purifying the organic acid from the medium. Purification can be performed by known techniques, such as the use of an ion exchange resin, activated carbon, microfiltration, ultrafiltration, nanofiltration, liquid-liquid extraction, crystallization, distillation, or chromatography, among others.

Preferably the yeast accumulates the organic acid in the medium during the culturing step, preferably the concentration of the organic acid is stabilized or allowed to increase.

In one embodiment of the invention, more than 10 g/I of the organic acid are accumulated in the culture medium. In a more preferred embodiment more than 30, 50, 70, 90 or 110 g/I of the organic acid are accumulated.

In a preferred embodiment of the invention, the gene increasing the hexose transport activity is selected from a hexose transporter gene or a gene coding for a protein influencing the hexose transport.

A hexose transporter gene is preferably isolated from *Saccharomyces cerevisiae.* Even more preferred the hexose transporter gene to be overexpressed in the yeast is selected from *HXT1, HXT2, HXT3, HXT4, HXT5, HXT6, HXT7, HXT8, HXT9, HXT10, HXT11, HXT12, HXT13, HXT14, HXT15, HXT16, HXT17, GAL2.*

A protein influencing the hexose transport can be a regulator or a glucose sensor or a gene required for proper expression of hexose transporters. The gene coding for a protein influencing the hexose transport is preferably isolated from *Saccharomyces cerevisiae* and even more preferably selected from *SNF3, GSF2* and *RGT2*.

In another embodiment the present invention the hexose transporter gene comprises one sequence identical with or corresponding to and having the functional characteristics of a sequence selected from the group consisting of SEQ ID NO 6 or 7, or a functionally equivalent variant of any of the foregoing sequences.

In another embodiment of the present invention additionally to the first hexose transporter a second hexose transporter is overexpressed in the yeast.

The invention provides a recombinant yeast with an improved organic acid production rate. In order to construct such a yeast, at least one DNA construct which comprises at least one gene encoding a protein facilitating the uptake of hexoses is introduced into the yeast, whereupon the gene encoding a protein facilitating the uptake of hexoses is functionally expressed. Preferably the recombinant yeast comprises at least one further recombinant gene encoding a protein related to the production of the desired organic acid.

One embodiment of the invention is a yeast strain that accumulates an organic acid in the culture medium and overexpresses at least one hexose transporter.

A further embodiment of the invention is a method to improve the productivity of an organic acid by a recombinant yeast strain by an increase of hexose uptake activity. The activity can be enhanced as outlined before, particularly by overexpression of one or more hexose transporters.

The following definitions are provided for the sake of a better interpretation of invention.

**"Sugar Transporter"** refers to any protein (e.g., enzyme), which can translocate a sugar, such as glucose, maltose, arabinose, across the plasma membrane. A sugar transporter can be a member of the 12 transmembrane domain superfamily showing this facilitating activity.

**"Hexose Transporter"** refers to any protein (e.g., enzyme), which can translocate a hexose such as glucose or fructose across the plasma membrane. A hexose transporter can be a member of the major facilitator superfamily. For the yeast *S. cerevisiae* at the time being 20 hexose transporters and associated proteins have been described, namely *HXT1* to *HXT17, GAL2, SNF3,* and *RGT2.*

An **"organic acid"** is an organic compound (i.e. any member of a large class of chemical compounds whose molecules contain carbon) with acidic properties. The most common organic acids are the carboxylic acids whose acidity is associated with their carboxyl group -COOH. Generally, organic acids are weak acids and do not dissociate completely, compared with strong mineral acids. Examples are acetic, lactic, succinic, citric, ascorbic, adipic acids.

**"Wild type yeast"** refers to a yeast, which when it has heritable genetic alterations introduced into its genome, results in the production of a mutant yeast. Restated, the mutant yeast strain has a different genotype than its wild type strain in that certain mutations, deletions or insertions have been introduced into its genome that are not present in the wild type yeast strain's genome. Thus, the wild type yeast strain lacks the changes that are present in the genome of the mutant yeast strain. The mutant yeast strain can, in some cases, have a different phenotype than the wild type strain. The mutant yeast strain can be prepared by methods known in the art, including those involving homologous recombination, directed mutagenesis or random mutagenesis, among others. In certain cases, the mutant yeast strain can be recovered by a process involving natural selection.

A **"recombinant yeast"** is a yeast that contains a nucleic acid sequence not naturally occurring in the yeast or an additional copy or copies of an endogenous nucleic acid sequence, wherein the nucleic acid sequence is introduced into the yeast or an ancestor cell thereof by human action. Recombinant DNA techniques are well-known, such as in Sambrook et al., Molecular Genetics: A Laboratory Manual, Cold Spring Harbor Laboratory Press, which provides further information regarding various techniques known in the art and discussed herein. In this embodiment, a coding region of the homologous and/or heterologous gene is isolated from an organism, which possesses the gene. The organism can be a bacterium, a prokaryote, a eukaryote, a microorganism, a fungus, a plant, or an animal.

Genetic material comprising the coding region can be extracted from cells of the organism by any known technique. Thereafter, the coding region can be isolated by any appropriate technique. In one known technique, the coding region is isolated by, first, preparing a genomic DNA library or a cDNA library, and second, identifying the coding region in the genomic DNA library or cDNA library, such as by probing the library with a labelled nucleotide probe selected to be or presumed to be at least partially homologous with the coding region, determining whether expression of the coding region imparts a detectable phenotype to a library microorganism comprising the coding region, or amplifying the desired sequence by PCR. Other known techniques for isolating the coding region can also be used.

**"Carbon source"** refers to an organic compound (e.g., defined carbon source, such as glucose, among others) or a mixture of organic compounds (e.g., yeast extract), which can be assimilated by a microorganism (e.g., yeast) and used to make new cell material. A mixture of organic compounds can be either a complex carbon source in which the exact components and/or the quantities of organic components are unknown or a defined carbon source that consists of known organic compounds (e.g., glucose, fructose, maltose, among others) in known quantities. In certain instances, a complex carbon source can also serve as a complex nitrogen source. Examples of complex carbon sources include starch, maltodextrose, cellulose hydrolysates, and starch hydrolysates, among others, which have been combined with enzymes to produce glucose. A defined carbon source will preferably be at least about 90 wt % pure, more preferably about 95 wt % pure, and most preferably about 98 wt % pure. For example, if glucose is the sole carbon source, the carbon source will comprise at least about 90% glucose. If glucose and fructose are the components of a defined carbon source, at least about 90% of the carbon source will be glucose and fructose. The defined carbon source will preferably comprise a minimal amount of higher saccharides.

**"Biomass"** refers to the whole cellular content derived from the biotransformation of the nutrients supplied to the cells.

**"Minimal media"** or "synthetic media" refers to culture media for culturing a microorganism (e.g., yeast) that comprise a nitrogen source, salts, trace elements, vitamins, and a carbon source, which are all defined. The carbon source can comprise at least one of glucose, sucrose, lactose, maltose, galactose, or fructose, among others. A minimal medium comprises non-protein nitrogen source. Synthetic media do not comprise for example, a nutrient source, whose composition is not defined, such as corn steep liquor, yeast extract or peptone, among others, which can be used in complex culture media.

**"Culturing in a liquid medium"** refers to growth of a microorganism and/or continued accumulation of an organic acid produced by a microorganism in a liquid culture medium.

**"Fermentation broth"** refers to a broth that is produced when a microorganism (e.g., yeast) is cultured in a liquid fermentation medium. The fermentation broth comprises any unused components of the liquid fermentation medium and any metabolites or products that result from fermentation by the organism.

**"Chemostat"** refers to a device that allows for a continuous culture of microorganisms (e.g., yeast) in which both specific growth rate and cell number can be controlled independently. A continuous culture is essentially a flow system of constant volume to which medium is added continuously and from which continuous removal of any overflow can occur. Once such a system is in equilibrium, cell number and nutrient status remain constant, and the system is in a steady state. A chemostat allows control of both the population density and the specific growth rate of a culture through dilution rate and alteration of the concentration of a limiting nutrient, such as a carbon or nitrogen source.

It is known in the art that chemostats can be used in selection of mutants of microorganisms. By altering the conditions as a culture is grown in a chemostat (e.g., decreasing the concentration of a secondary carbon source necessary to the growth of the inoculum strain, among others) those microorganisms in the population that are capable of growing faster at the altered conditions will be selected and outgrow microorganisms that do not function as well under the new conditions. Typically such selection requires the progressive increase or decrease of at least one culture component over the course of growth of the chemostat culture.

**"Batch culture"** refers to a closed culture of microorganisms with growth occurring in a fixed volume of culture medium that is continually being altered by the actions of the growing organisms until it is no longer suitable for growth. In batch culture, all nutrients required for microbial growth are present in the medium before beginning cultivation, except for molecular oxygen in aerobic cultivation. It is known in the art that extended cultivation of microorganisms in batch cultures (e.g., shake flasks) can be used to select for spontaneous mutants that grow relatively well under conditions in which the inoculum strain would not grow, or grows poorly.

A **"fed-batch cultivation"** refers to a culturing technique in which one or more nutrients are supplied into the culture medium in a cultivation vessel or fermenter over the course of cultivation of a microorganism. In contrast to a chemostat culture, the microorganisms are contained during cultivation. In some cases, all nutrients are gradually fed to the fermenter. The time conditions, temperature conditions, pH conditions, aeration conditions, and the rate at which certain nutrients are fed to a fermenter depend on the particular strain that is being used.

**"Productivity"** refers to the quantity of product derived from production processes, per unit of time.

**"Selection"** refers to placing yeast under conditions that favour the growth of cells having a particular genotype or particular genotypes. The particular genotype (often the result of a genetic mutation) confers upon the selected yeast an advantage under certain environmental conditions so that the progeny of the selected yeast are able to outgrow and/or replace the parent.

The term **"gene"** refers to chromosomal DNA, plasmid DNA, cDNA, synthetic DNA, or other DNA that encodes a peptide, polypeptide, protein, or RNA molecule, and regions flanking the coding sequence involved in the regulation of expression.

**"Mutation"** refers to any change or alteration in a nucleic acid sequence. Several types exist, including point, frame shift, and splicing. Mutation may be performed specifically or randomly.

**"Open reading frame (ORF)"** refers to a region of DNA or RNA encoding a peptide, polypeptide, or protein.

The term **"promoter"** or "promoter region" refers to a DNA sequence that includes elements controlling the production of messenger RNA (mRNA) by providing the recognition site for RNA polymerase and/or other factors necessary for start of transcription at the correct site.

**"Plasmid"** refers to a circular, extrachromosomal, self-replicating piece of DNA.

The term "genome" encompasses both the chromosome(s) and plasmids within a host cell.

A **"Vector"** is a small piece of DNA. A vector functions like a "molecular carrier", which will carry desired fragments of DNA into a host cell. Foreign fragments of DNA may be inserted into the vector, they may or may not contain regulatory and coding sequences of interest, an origin of replication, which enables the vector, together with the foreign DNA fragment inserted into it, to replicate, genetic markers that allow for selection of cells which have taken up the plasmid DNA and other features. A vector may be a plasmid or a linear DNA molecule.

**"Transcription"** refers to the process of producing a complementary RNA from a DNA template.

**"Translation"** refers to the production of protein from messenger RNA.

The term **"expression"** refers to the transcription of a gene to produce the corresponding mRNA and translation of this mRNA to produce the corresponding gene product, i.e., a peptide, polypeptide, or protein.

**"Overexpression"** refers to a level of expression of a given gene, greater than the level of expression in a wild-type yeast.

The phrase **"functionally linked"** or **"operably linked"** refers to a promoter or promoter region and a coding or structural sequence in such an orientation and distance that transcription of the coding or structural sequence may be directed by the promoter or promoter region.

**"Heterologous DNA"** refers to DNA from a source different than that of the recipient cell.

**"Homologous DNA"** refers to DNA from the same source as that of the recipient cell.

**Example 1:** Construction of *S. cerevisiae* (GRF18U and CEN.PK) strains (over)expressing the *HXT1* or *HXT7* genes: cellular growth, glucose consumption and ethanol production of transformants and control strains.

The *S. cerevisiae HXT1* and *HXT7* genes were PCR amplified using as a template the genomic DNA extracted from the GRF18U strain.

The oligos for the amplification are the following:
**5'HXT1 *(SEQ ID NO.: 1)*** 5'-AAA ATC ATG AAT TCA ACT CCC GAT CTA -3' Tm: 58.9
**3'HXT1 *(SEQ ID NO.: 2)*** 5'-AGC TTG TTT AGT TTA TTT CCT GCTG AAA -3' Tm: 59.3
**5'HXT7 *(SEQ ID NO.: 3)*** 5'-A AAA ATG TCA CAA GAC GCT GCT ATT GCA -3' Tm: 62.4
**3'HXT7 exit *(SEQ ID NO.: 4)*** 5'-ATA TAT TAA AAA CGT ATT TAC TTT TCA AGT -3' Tm: 54.2
**3'HXT7 *(SEQ ID NO.: 5)*** 5'-AGT GTC GAC AAA TAA TTT GGT GCT GAA CAT-3' Tm: 61.0

The following program was used for all the amplifications:

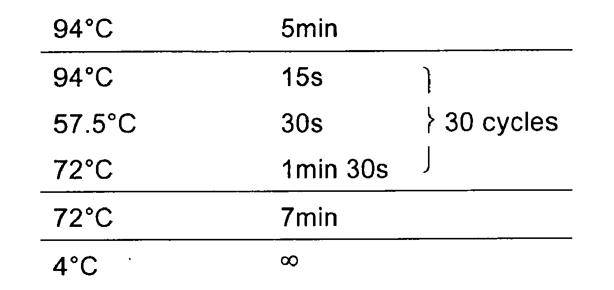

Because of the high sequence omology between the coding sequence of the *S. cerevisiae HXT6* and *HXT7* genes, the latter was amplified in two steps. In the first step the oligos named 5'HXT7 and 3'HXT7exit were used, the second designed on an external region in respect to the gene that resulted different from the corresponding region of the *HXT6* gene. The single amplified band was secondarily used as a template for the two oligos 5'HXT7 and 3'HXT7, obtaining the sole open reading frame of the desired gene.

The amplified fragments were subcloned in the *E. coli* vector pSTBlue obtaining, respectively, the plasmids pSTBlueHXT1 and pSTBlueHXT7. The inserts were sequenced and resulted identical to the deposited *S. cerevisiae* corresponding sequences (*HXT1,* GeneID: 856494, SEQ ID NO:6 and *HXT7,* GeneID: 851943, SEQ ID NO:7).

These coding sequences are used for the construction of the integrative expression plasmids p022HXT1 and p022HXT7, respectively, utilizing the basic *S. cerevisiae* integrative expression plasmid pYX022 (R&D Systems, Inc., Wiesbaden, D).

For the construction of the plasmid p022HXT1, the recipient vector was *Eco*RI cut, blunted and dephosphorylated, while the insert was *Mlu*I/*Pml*I excised from the pSTBlueHXT1 plasmid. For the construction of the plasmid p022HXT7, the recipient and the pSTBlueHXT7 vectors were *Eco*RI cut.

DNA manipulation, transformation and cultivation of *E. coli* (Novablue, Novagen), were performed following standard protocols (Sambrook J., et al., Molecular Cloning: A Laboratory Manual, 2nd edn., Cold Spring Harbor Laboratory, New York, 1989). Also other basic molecular biology protocols were performed following this manual if not otherwise stated. All the restriction and modification enzymes utilised are from NEB (New England Biolabs, UK) or from Roche Diagnostics.

Transformation of both the *S. cerevisiae* strains was performed basically according to the LiAc/PEG/ss-DNA protocol (Gietz. 2002). The transformation with the obtained integrative plasmids was favoured by linearising them in the auxotrophic marker *HIS3.* The transformants were plated on selective minimal medium and single colonies were obtained after 3-4 days of growth at 30°C.

In order to check the effect of the HXT overexpression, independent transformants (transformed with the plasmid harbouring the *HXT1* or the *HXT7* gene) and respective controls were shake flask cultured in minimal medium (YNB, 1.34% w/V YNB from Difco Laboratories, Detroit, MI #919-15, 2% w/V Glucose, complemented with Uracil or with Uracile and Leucine.
Figures 1 and 2 show on the left the cell density (OD 660nm) and on the right the glucose consumption and the ethanol accumulation during the kinetics of growth of the GRF18U and of the CEN.PK strains, respectively. The glucose consumption and the ethanol accumulation were determined using a commercially available enzymatic kit from Megazyme (cat. n. K-GLUHKL and K-ETOH, respectively), according to the manufacturer's instructions. During the kinetics, samples were collected and the described parameters determined at the indicated times (see "hours" of Figures 1 and 2).

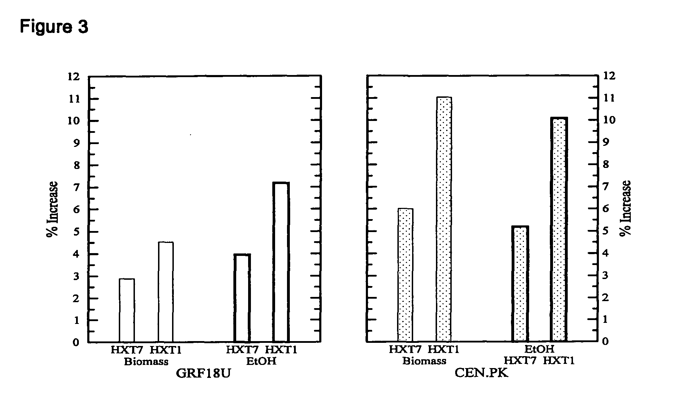

The experiments performed clearly show that the presence of an additional copy of an hexose transporter under the control of a glycolytic promoter determines a quicker uptake of the sugar (as it can be argued from the glucose consumption curves, Figures 1b and 2b, open symbols) and a quicker and higher ethanol production (Figures 1b and 2b, full symbols). Remarkably, the described effect is confirmed in more than one yeast background (Figures 1 and 2). Surprisingly, the effect of a higher glycolytic flux appears with a very similar kinetic independently from the hexose transporter overexpressed, despite a slightly higher effect is reached by *HXT1* overexpression, as hypotesized. Moreover, it is very interesting to underline that depending on the yeast background, the effect of the higher glycolytic flux is distributed differently on the fermentation products or on the biomass accumulation, as shown in Figure 3 where the two strains are compared. Finally, it is worth to consider not only the improvement in ethanol production and/or in biomass formation, but also the specific productivity, in other words, the reduced time needed to reach higher values of production.

**Example 2:** Construction of *S. cerevisiae* (GRF18U and CEN.PK) strains expressing a heterologous LDH activity from *Lactobacillus plantarum* and (over)expressing the *HXT1* or *HXT7* genes; cellular growth, glucose consumption, lactate and ethanol production of transformants and control strains.

The *S. cerevisiae* strains (GRF18U and CEN.PK) already transformed with the p022HXT1 and p022HXT7 expression vector were further transformed with the plasmid named Ycp111*b*TLDH. Said plasmid was obtained by inserting the LDH gene under the control of the *Z. bailii* TPI promoter in the basic *S. cerevisiae* centromeric plasmid Ycplac111 (*LEU2* marker, AC X75457). To obtain said plasmid, the *L. plantarum* LDH gene was previously PCR amplified and subcloned in the *E. coli* vector pSTBlue, resulting in the pSTpILDH plasmid (Microb Cell Fact. 2006 Jan 30;5:4. Lactate production yield from engineered yeasts is dependent from the host background, the lactate dehydrogenase source and the lactate export. Branduardi P, Sauer M, De Gioia L, Zampella G, Valli M, Mattanovich D, Porro D.). Both the *E. coli* construct and a basic *Z. bailii* centromeric expression plasmid pZ₃*b*T (Branduardi et al. 2004) were *EcoR*I cut, the latter being also dephosphorylated, to obtain the yeast expression vector pZ₃*b*TLDH. From said plasmid the expression cassette, comprising the LDH coding sequence and the yeast promoter and terminator regions, was *BamH*I blunt/ *Nae*I excised and inserted in the recipient Ycplac111 plasmid, opened Smal and de-phosphorilated, resulting in the Ycp111*b*TLDH centromeric expression plasmid that was then used for transforming the above mentioned yeast strains. The control strain is, for each background, the corresponding yeast strain transformed with the sole LDH gene, together with the empty plasmid used for the HXT (over)expression.

Transformation of both the *S. cerevisiae* strains was performed basically according to the LiAc/PEG/ss-DNA protocol (Technical Tips Online, http://tto.trends.com). The transformants were plated on selective minimal medium and single colonies were obtained after 3-4 days of growth at 30°C.

Independent transformants were shake flask cultured in minimal medium (YNB, 1.34% w/V YNB from Difco Laboratories, Detroit, MI #919-15, 5% w/V Glucose, Figures 4.1-4.2 and 5.1-5.2). The LDH expression determines that the transformant yeasts will ferment the pyruvate not only to ethanol but also to lactate: the co-expression of the *HXT* gene determines an improvement in the accumulation of the fermentation products, in both strains. The experiments reported in the following figures refer to the co-expression of the *L. plantarum* LDH with the *S. cerevisiae HXT1* or *HXT7* genes. Figures 4 and 5a shows the cell density (OD 660nm) reached by the different clones and constructs, where Figures 4 and 5 b, c and d show the glucose consumption and the ethanol and lactate production, respectively, during the kinetics of growth (samples were collected at the indicated times, see "hours" on the Figures). The glucose consumption as well as the ethanol and lactate production was determined using commercially available enzymatic kits from Megazyme (cat. n. K-GLUHKL, K-ETOH and K-LATE, respectively), according to the manufacturer's instructions. Remarkably, also in these experiments the increased flux of glucose consumption results in a quicker and higher production of the fermentation products, being in this case the natural produced ethanol and the lactic acid produced thanks to the heterologous production of a bacterial LDH. It is important to notice that the two pathways seem to compete, so that the fraction of glucose redirect to fermentation is distributed between lactic acid and ethanol.

**Example 3:** Construction of *S. cerevisiae* (CEN.PK) strains expressing a heterologous LDH activity from *Lactobacillus plantarum* and (over)expressing the *HXT1* or the *HXT7* gene; cellular growth, glucose consumption, lactate and ethanol production of transformants and control strains.

In this example the lactate and ethanol production of a CEN.PK strain transformed with a multicopy *L. plantarum* LDH was compared to the production obtained with an additional copy of the *HXT1* or of the *HXT7* gene.

The bacterial lactate dehydrogenase was *EcoR*I excised from the previously mentioned pSTpILDH and inserted in the similarly cut and dephosphorylated plasmid pYX212 (the basic *S. cerevisiae* multicopy expression plasmid pYX212, *LEU2* marker, R&D Systems, Inc., Wiesbaden, D), resulting in the expression plasmid p212IpLDH. Independent transformants derived from the yeast transformation were shake flask cultured in minimal medium (YNB, 1.34% w/V YNB from Difco Laboratories, Detroit, MI #919-15, 5% w/V Glucose).

Figures 6.1 and 6.2 show on the left the cell density (OD 660nm) and the glucose consumption, on the right the ethanol and lactate accumulation during the kinetics of growth of the CEN.PK transformed strains. The glucose consumption as well as the ethanol and lactate accumulation was determined using a commercially available enzymatic kit from Megazyme (cat. n. K-GLUHKL, K-ETOH and K-LATE, respectively), according to the manufacturer's instructions. During the kinetics, samples were collected and the described parameters determined at the indicated times (see "hours" of Figures 6.1 and 6.2). Remarkably, while with the expression of an additional copy of *HXT*1 both the ethanol and the lactic acid production result improved, with the expression of an additional copy of *HXT7* the increase in the lactic acid production corresponds to a parallel decrease in the ethanol production. Moreover, in this last example is once more evident an increase in productivity, since the highest value in lactic acid production is reached earlier than in the control strain. Finally, it can be noticed, in respect to the experiment performed with the *LDH* expressed in a centromeric plasmid, that the effect of the *LDH* expressed by a multicopy plasmid is significantly more effective.

**Example 4:** Lactic acid production in the m850 (*S. cerevisiae* CEN.PK, *pdc, LpLDH*) strain (over)expressing the *HXT1* or the *HXT7* gene.

The phototrophic CEN.PK m850 strain, totally impaired for the pyruvate decarboxylase activity and bearing the *L. plantarum* LDH in a

multicopy yeast expression plasmid, was transformed to express an additional copy of the *HXT1* or of the *HXT7* gene. For the obtainment of said transformants, a yeast integrative plasmid harbouring an auxotrophic marker used only as a target gene and a dominant marker used for the effective selection of the transformants was built. The backbone of the plasmid was the basic *S. cerevisiae* integrative expression plasmid pYX022, *HIS3* marker (R&D Systems, Inc., Wiesbaden, D). Said plasmid was *Kpn*I cut, blunted and dephosphorylated and ligated with the KanR cassette derived *Sph*I/*Sac*I-blunt from the plasmid pFA6KanMX4 (Wach, *et al*., 1994), resulting in the plasmid p022KMX4. Said plasmid was *EcoR*I cut and dephosphorylated or *EcoR*I cut, blunt and dephosphorylated and ligated with the *HXT1* and *HXT7* sequences, respectively, prepared as described in Example 1. The resulting plasmids were named p022KMX4-1 and p022KMX4-7, respectively.

The m850 strain was then transformed with these two plasmids as well as with the empty one. Independent transformants were then tested for glucose consumption and lactate production (the m850 strain can not produce ethanol because of the impairment of all the *pdc* activities, and for the same reason they can not live on glucose as a carbon source, but they are viable on non fermenting carbon sources, such as ethanol and glycerol, that were actually used for all the phases of this strain manipulation), as shown in Figure 7.

The transformants were grown as follows: they were propagated on agar plates containing 1.7 g/l yeast nitrogen base (YNB) without amino acids, 10 g/l ethanol, and 10 g/l glycerol. Two different liquid media were used to cultivate the strains: a preinoculum medium containing 0.31 g/l CaCO₃, 1.7 g/l YNB without amino acids and without (NH₄)₂SO₄, 1.5 g/l urea, 10 g/l ethanol, and 0.5 g/l glucose and a fermentation medium containing 4.5 g/l CaCO₃, 1.7 g/l YNB without amino acids and without (NH4)2SO4, 1 g/l urea, 5 g/l ethanol, and 90 g/l glucose, as specified. Batch culture was performed at 28°C in 250-ml quadruple baffled shake flasks. The cells were harvested from fresh cultures grown on agar plates and used to inoculate 100 ml of preinoculum medium at an optical density at 600 nm (OD600) of 0.3. After 24 h these cells were harvested and used to inoculate 100 ml of fermentation medium at an OD600 of 3.0. Batch cultures were monitored for approximately 70 h.

At indicated times, samples were collected and the OD 660 nm was measured (Figure 7a), while the supernatants were tested for glucose consumption (Figure 7b) and lactic acid production (Fig. 7c).

Remarkably, the effect of the hexose transporter overexpression results in a quicker glucose consumption (at least for the strains overepressing an additional copy of the *HXT*1 gene) and in a consequent quicker production of the sole fermentation product possible in this strain, the lactic acid, resulting in an improved productivity. This experiment demonstrates the possibility to apply what previously shown in laboratory strains also in a strain already developed and optimised for industrial productions.

### References

Branduardi P, Valli M, Brambilla L, Sauer M, Alberghina L, Porro D. (2004) The yeast Zygosaccharomyces bailii: a new host for heterologous protein production, secretion and for metabolic engineering applications. FEMS Yeast Res. 4, 493-504.
Gietz, R.D. and R.A. Woods. (2002) Transformation of yeast by the liac/ss carrier dna/peg method. Methods in Enzymology 350: 87-96.
Perez, M., Luyten, K., Michel, R., Riou, C., Blondin, B. (2005) Analysis of Saccharomyces cerevisiae hexose carrier expression during wine fermentation: both low- and high-affinity Hxt transporters are expressed. FEMS Yeast Research 5, 351-361.
Guillaume C, Delobel P, Sablayrolles JM, Blondin B. (2007) Molecular basis of fructose utilization by the wine yeast Saccharomyces cerevisiae: a mutated HXT3 allele enhances fructose fermentation. Appl Environ Microbiol. 73, 2432-9.
Gutiérrez-Lomelí M, Torres-Guzmán JC, González-Hernández GA, Cira-Chávez LA, Pelayo-Ortiz C, Ramírez-Córdova Jde J..(2008) Overexpression of ADH1 and HXT1 genes in the yeast Saccharomyces cerevisiae improves the fermentative efficiency during tequila elaboration. Antonie Van Leeuwenhoek. 93, 363-71.
Wach A, Brachat A, Pohlmann R and Philippsen P (1994) New heterologous modules for classical or PCR-based gene disruptions in Saccharomyces cerevisiae. Yeast 10, 1793-1808.

## Claims

1. A method for production of an organic acid comprising:
a) culturing a yeast strain, that
i) overexpresses at least one sugar transporter, and
ii) accumulates an organic acid in the culture medium, and
b) purification of the organic acid.

2. A method according to claim 1, comprising:
a. obtaining a recombinant yeast with increased sugar transporter activity;
b. culturing the recombinant yeast in a medium comprising a sugar, thereby forming the organic acid, and
c. isolating the organic acid.

3. A method according to claim 1 or 2 wherein said sugar transporter activity is an hexose transporter activity and said sugar is an hexose.

4. The method according to claims 1-3, wherein the recombinant yeast overexpresses at least one hexose transporter.

5. The method according to anyone of claims 1 to 4, wherein the organic acid that is produced is selected from the group consisting of lactic acid, itaconic acid, succinic acid, citric acid, maleic acid.

6. The method according to anyone of claims 1 to 5, wherein the yeast strain overexpresses at least one further gene related to the organic acid production.

7. The method according to anyone of claims 1 to 6, wherein the yeast strain is a strain of *Saccharomyces, Zygosaccharomyces, Pichia or Kluyveromyces.*

8. The method according to anyone of claims 1 to 7, wherein the hexose transporter gene is selected from the group consisting of *HXT1, HXT2, HXT3, HXT4, HXT5, HXT6, HXT7, HXT8, HXT9, HXT10, HXT11, HXT12, HXT13, HXT14, HXT15, HXT16, HXT17, GAL2, SNF3,* and *RGT2.*

9. The method according to anyone of claims 1 to 8, wherein more than 10 g/I of the organic acid are accumulated.

10. The method according to claim 8 , wherein the gene for the hexose transporter is isolated from *Saccharomyces cerevisiae.*

11. A yeast strain that accumulates an organic acid in the culture medium and overexpresses at least one sugar transporter, preferably a hexose transporter.

12. A method to improve the productivity of an organic acid by a recombinant yeast strain by an increase of sugar uptake activity, preferably hexose transport activity.

13. A method to improve the production of biomass by an increase of sugar uptake activity, preferably hexose transport activity.

14. The method according to claim 12, wherein the increase of the hexose uptake activity is caused by the overexpression of a hexose transporter.


SEQUENCE LISTING

<110>  Università degli Studi Milano-Bicocca

<120>  Improved yeast strains for organic acid production

<130>  2035EUR

<160>  7

<170>  PatentIn version 3.3

<210>  1
<211>  27
<212>  DNA
<213>  Artificial

<220>
<223>  primer

<400>  1
aaaatcatga attcaactcc cgatcta                                          27


<210>  2
<211>  28
<212>  DNA
<213>  artificial

<220>
<223>  primer

<400>  2
agcttgttta gtttatttcc tgctgaaa                                         28


<210>  3
<211>  28
<212>  DNA
<213>  Artificial

<220>
<223>  primer

<400>  3
aaaaatgtca caagacgctg ctattgca                                         28


<210>  4
<211>  30
<212>  DNA
<213>  Artificial

<220>
<223>  primer

<400>  4
atatattaaa aacgtattta cttttcaagt                                       30


<210>  5

<211>  30
<212>  DNA
<213>  Artificial

<220>
<223>  primer

<400>  5
agtgtcgaca aataatttgg tgctgaacat                                    30


<210>  6
<211>  1713
<212>  DNA
<213>  Saccharomyces cerevisiae

<400>  6
atgaattcaa ctcccgatct aatatctcct cagaaatcca attcatccaa ctcatatgaa      60

ttggaatctg gtcgttcaaa ggccatgaat actccagaag gtaaaaatga aagttttcac     120

gacaacttaa gtgaaagtca agtgcaaccc gccgttgccc ctccaaacac cggaaaaggt     180

gtctacgtaa cggtttctat ctgttgtgtt atggttgctt tcggtggttt catatttgga     240

tgggatactg gtaccatttc tggttttgtt gctcaaactg attttctaag aagatttggt     300

atgaagcacc acgacggtag tcattacttg tccaaggtga gaactggttt aattgtctct     360

atttttaaca ttggttgtgc cattggtggt atcgtcttag ccaagctagg tgatatgtat     420

ggtcgtagaa tcggtttgat tgtcgttgta gtaatctaca ctatcggtat cattattcaa     480

atagcctcga tcaacaagtg gtaccaatat ttcattggta gaattatctc tggtttaggt     540

gtcggtggta tcacagtttt atctcccatg ctaatatctg aggtcgcccc cagtgaaatg     600

agaggcacct tggtttcatg ttaccaagtc atgattactt taggtatttt cttaggttac     660

tgtaccaatt ttggtaccaa gaattactca aactctgtcc aatggagagt tccattaggt     720

ttgtgtttcg cctgggcctt atttatgatt ggtggtatga tgtttgttcc tgaatctcca     780

cgttatttgg ttgaagctgg cagaatcgac gaagccaggg cttctttagc taaagttaac     840

aaatgcccac ctgaccatcc atacattcaa tatgagttgg aaactatcga agccagtgtc     900

gaagaaatga gagccgctgg tactgcatct tggggcgaat tattcactgg taaaccagcc     960

atgtttcaac gtactatgat gggtatcatg attcaatctc tacaacaatt aactggtgat    1020

aactatttct tctactacgg taccattgtt ttccaggctg tcggtttaag tgactctttt    1080

gaaacttcta ttgtctttgg tgtcgtcaac ttcttctcca cttgttgttc tctgtacacc    1140

gttgaccgtt ttggccgtcg taactgtttg atgtggggtg ctgtcggtat ggtctgctgt    1200

tatgttgtct atgcctctgt tggtgttacc agattatggc caaacggtca agatcaacca    1260

tcttcaaagg gtgctggtaa ctgtatgatt gttttcgcat gtttctacat tttctgtttc    1320

gctactacct gggccccaat tgcttacgtt gttatttcag aatgtttccc attaagagtc 1380

aaatccaagt gtatgtctat tgccagtgct gctaactgga tctgggtttc cttgattagt 1440

ttcttcaccc catttattac tggtgccatc aacttctact acggttacgt tttcatgggc 1500

tgtatggttt tcgcttactt ttacgtcttt ttcttcgttc cagaaactaa aggtttatca 1560

ttagaagaag ttaatgatat gtacgccgaa ggtgttctac catggaaatc agcttcctgg 1620

gttccagtat ccaagagagg cgctgactac aacgctgatg acctaatgca tgatgaccaa 1680

ccattttaca agagtttgtt tagcaggaaa taa 1713

<210> 7
<211> 1713
<212> DNA
<213> Saccharomyces cerevisiae

<400> 7
atgtcacaag acgctgctat tgcagagcaa actcctgtgg agcatctctc tgctgttgac 60

tcagcctccc actcggtttt atctacacca tcaaacaagg ctgaaagaga tgaaataaaa 120

gcttatggtg aaggtgaaga gcacgaacct gtcgttgaaa ttccaaagag accagcttct 180

gcctatgtca ctgtctctat tatgtgtatc atgatcgcct ttggtggttt cgttttcggt 240

tgggatactg gtaccatttc tggtttcatc aatcaaaccg atttcatcag aagatttggt 300

atgaagcata aagatggtac taattatttg tctaaggtta gaactggttt gattgtctcc 360

attttcaaca ttggttgtgc cattggtggt attattcttt ccaaattggg tgatatgtac 420

ggtcgtaagg tgggtttgat tgtcgttgtt gtcatctaca tcatcggtat tattattcaa 480

attgcatcta tcaacaaatg gtaccaatat ttcatcggta gaattatttc cggtttgggt 540

gttggtggta ttgccgtttt atctcctatg ttgatttctg aagtatcccc aaagcattta 600

aggggtactt tagtctcttg ctaccaattg atgattactg ccggtatttt cttgggttac 660

tgtaccaact tcggtactaa gaactactcc aactctgtgc aatggagagt tccattaggt 720

ttgtgttttg cctgggcttt gtttatgatt ggtggtatga catttgttcc agagtctcca 780

cgttatttgg ctgaagtcgg taagatcgaa gaagccaaac gttctattgc cgtttctaac 840

aaggttgctg ttgatgatcc atctgttttg gctgaagtcg aagctgtctt ggctggtgta 900

gaggcagaga aattagctgg taatgcatcc tggggtgaat tgtttagtag caagacaaag 960

gtccttcagc gtttgatcat gggtgctatg attcaatctc tacaacaatt gacaggtgat 1020

aactatttct tctactatgg tactactatt ttcaaggctg ttggtttgag tgactctttc 1080

gaaacctcta ttgtcttggg tattgttaac tttgcttcca cctttgttgg tatttacgtt 1140

gttgagagat atggtcgtcg tacttgtttg ctatggggtg ctgcatccat gactgcttgt 1200

atggttgtct atgcttccgt gggtgtcacc agattatggc caaatggtca agaccaacca 1260

tcttccaagg gtgctggtaa ctgtatgatt gtctttgcct gtttctatat tttctgtttt 1320

gctactacat gggctccaat tccttatgtc gttgtttctg aaactttccc attgagagtc 1380

aagtctaagg ctatgtctat tgctacagct gctaattggt tgtggggttt cttgattggt 1440

ttcttcactc catttattac tggtgctatt aacttctact acggttacgt tttcatgggc 1500

tgtttggtct tcatgttctt ctatgttttg ttagttgttc cagaaactaa gggtttgact 1560

ttggaagaag tcaacaccat gtgggaagaa ggtgttctac catggaagtc tgcctcatgg 1620

gttccaccat ccagaagagg tgccaactac gacgctgaag aaatgactca cgatgacaag 1680

ccattgtaca agagaatgtt cagcaccaaa taa 1713